# EUROPEAN PATENT SPECIFICATION

(11) **EP 1 875 931 B1**
(45) Date of publication and mention of the grant of the patent: **24.08.2011**
(21) Application number: 06728685.6
(22) Date of filing: 07.03.2006
(51) Int. Cl.: A61M 1/14, A61M 1/36

(54) **BLOOD DEPURATOR**
BLUTDEPURATOR
EPURATEUR DE SANG

(30) Priority: 28.03.2005 JP 2005091983
(43) Date of publication of application: 09.01.2008
(73) Proprietor: Nikkiso Company Limited, Tokyo 150-0013 (JP)
(72) Inventor: NIMURA, Hiroshi, ohara-shi, Shizuoka, 4210496 (JP); MORI, Azusa, ohara-shi, Shizuoka, 4210496 (JP); UEDA, Yoshiro, ohara-shi, Shizuoka, 4210496 (JP)
(74) Representative: GROSSE SCHUMACHER KNAUER VON HIRSCHHAUSEN
(86) International application number: PCT/JP2006/304316
(87) International publication number: WO 2006/103883

(56) References cited:
- EP-A1- 1 457 218
- JP-A- 2004 187 990
- US-A- 6 132 616
- CAVANAUGH D J ET AL: "Hemodialysis blood transport system" IBM TECHNICAL DISCLOSURE BULLETIN, INTERNATIONAL BUSINESS MACHINES CORP. (THORNWOOD), US, vol. 19, no. 3, 1 August 1976 (1976-08-01) , pages 765-768, XP002124307 ISSN: 0018-8689

## Description

### TECHNICAL FIELD

The present invention relates to a blood purification apparatus, which purifies the blood of a patient by circulating the blood extracorporeally in a dialysis treatment using a dialyzer or the like.

### BACKGROUND ART

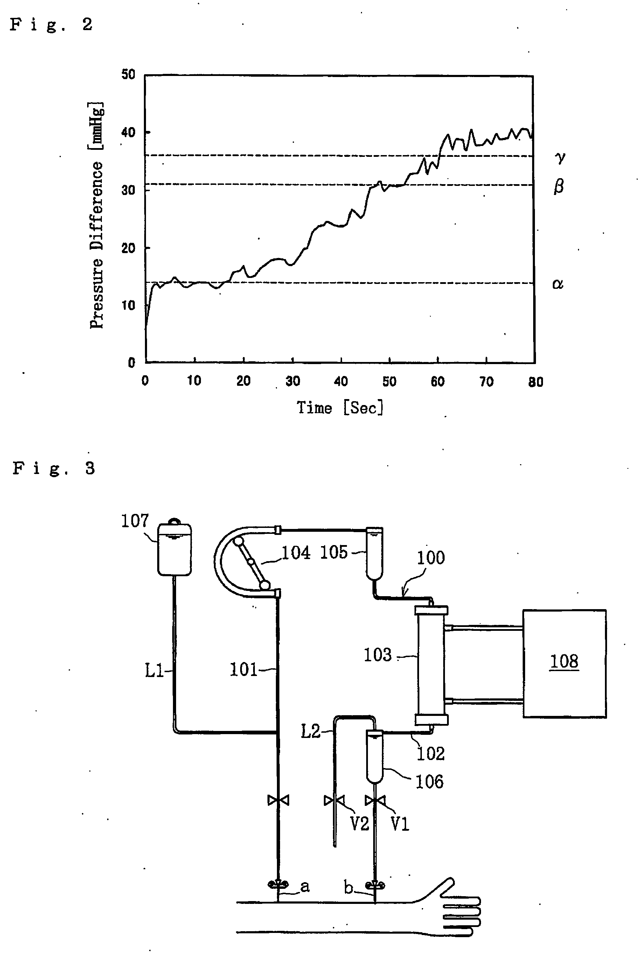

As shown in Fig. 3, a dialysis apparatus in a blood purification apparatus primarily includes a blood circuit 100 formed by an arterial blood circuit 101 with an arterial needle "a" and a venous blood circuit 102 with a venous needle "b", a dialyzer 103 for purifying a blood flowing through the blood circuit 100 between the arterial blood circuit 101 and the venous blood circuit 102, a blood pump 104 provided at the arterial blood circuit 101, an arterial drip chamber 105 and a venous drip chamber 106 provided in the arterial blood circuit 101 1 and the venous blood circuit 102, respectively, an overflow line L2 extended from an air-layer side of the venous drip chamber 106, and a dialysis device 108 for supplying dialysate to the dialyzer 103.

In addition, a saline solution bag 107 supplied with a priming solution (saline solution) through a saline line L1 is connected to a portion of the arterial blood circuit 101 between the arterial needle "a" and the blood pump 104, which can perform priming prior to a dialysis treatment and supply an additional priming solution during the dialysis treatment. For example, when rinsing and priming are performed, an end portion of the arterial blood circuit 101 and an end portion of the venous blood circuit 102 are connected to each other before the arterial needle "a" and the venous needle "b" are mounted on the circuits 101 and 102, respectively. Subsequently, the blood pump 104 is turned on, so that the blood circuit 100 is entirely filled with the priming solution while discharging the priming solution from the overflow line L2.

To start the dialysis treatment, the arterial needle "a" and the venous needle "b" are mounted on the end portions of the arterial blood circuit 101 and the venous blood circuit 102, respectively, and are inserted into the patient. Then, the blood pump 104 is driven while supplying the dialysate from the dialysis device 108 to the dialyzer 103, the blood of the patient is introduced into the blood circuit 100 through the arterial needle "a" and passes through the dialyzer 103 so that the blood is purified and water is removed therefrom. Then, the blood is returned into the patient through the venous needle "b". For instance, such a blood purification apparatus is discloses by Patent Document 1, which additionally offers the functionalities of controlling the back-filtration of the dialysate as well as a failure detection during the blood withdrawal process.

At the time the dialysis treatment is started, in order to prevent a large amount of the priming solution pre-filled in the blood circuit 100 from being introduced into the body of the patient through the venous needle "b", in the prior art, electromagnetic valves V1 and V2 are firstly set to be in closed and open states, respectively, and then are switched to be in open and closed states, respectively, when it is visually confirmed that the priming solution in the vicinity of the venous needle "b" is replaced with the blood.

Thus, the states of the electromagnetic valves V1 and V2 are switched, so that the priming solution is discharged from the overflow line L2 prior to replacement with the blood in the vicinity of the venous needle "b", and the blood which was replaced with the priming solution is returned to the body of the patient through the venous needle "b". The above-described prior art, which discharges the priming solution from the overflow line L2 and replaces the priming solution with the blood, is disclosed in Patent Document 2, for example.
Patent Document 1: EP 1 457 218 A1 Patent Document 2: JP-2002-325837-A

### SUMMARY OF THE INVENTION

### PROBLEMS TO BE SOLVED BY THE INVENTION

However, in the above-described prior art blood purification apparatus, since the electromagnetic valves V1 and V2 are manually switched after a medical staff (such as a medical doctor) visually confirms that the priming solution in the vicinity of the venous needle "b" is replaced with the blood, the medical staff has to carefully monitor the vicinity of the venous needle "b" at the time the dialysis treatment is started, making the dialysis treatment inefficient. Also, if the electromagnetic valves V1 and V2 are switched too early, a large amount of priming solution is introduced into the body of the patient. On the other hand, if the electromagnetic valves V1 and V2 are switched too late, the patient's extracorporeally-circulated blood is discharged from the overflow line L2, making a problem.

In view of the foregoing circumstances, the present invention is intended to provide a blood purification apparatus which can improve the operating of a blood purification treatment at the time the treatment is started, prevent a large amount of priming solution from being introduced into the body of a patient, and efficiently return the blood into the patient after extracorporeally circulating the blood.

### MEANS FOR SOLVING THE PROBLEM

The invention described in claim 1 is a blood purification apparatus characterized by comprising: a blood circuit formed by an arterial blood circuit and a venous blood circuit, the blood circuit being pre-filled with a priming solution in advance for extracorporeally-circulating the blood of a patient from an end portion of the arterial blood circuit to an end portion of the venous blood circuit; a blood purifying means, provided between the arterial blood circuit and the venous blood circuit of the blood circuit, for purifying the blood flowing through the blood circuit; a first valve provided for opening and closing the vicinity of the end portion of the venous blood circuit to prevent and allow the flow of the priming solution and the blood flowing into the patient through the end portion of the venous blood circuit; a discharge line extending from an upstream side of the first valve of the blood circuit for discharging the priming solution flowing through the blood circuit; and a second valve provided for opening and closing the discharge line to prevent and allow the flow of the priming solution and the blood flowing through the discharge line, wherein the vicinity of the end portion of the venous blood circuit on the upstream side of the first valve is provided with a detection means for detecting a replacement of the priming solution with the blood, and the detection of the replacement by the detection means automatically switches the first valve from a closed state to an open state and the second valve from an open state to a closed state.

The invention described in claim 2 is, in the blood purification apparatus described in claim 1, characterized in that the arterial blood circuit and the venous blood circuit are provided with an arterial drip chamber and a venous drip chamber, respectively, for removing bubbles in the priming solution and the blood, and the discharge line is constructed by an overflow line extending from an air-layer side of the venous drip chamber.

The invention described in claim 3 is, in the blood purification apparatus described in claim 2, characterized in that replacement of the priming solution with the blood is detected, based on a pressure difference between a liquid pressure in the arterial drip chamber and a liquid pressure in the venous drip chamber, in the vicinity of the end portion of the venous blood circuit on the upstream side of the first valve.

The invention described in claim 4 is, in the blood purification apparatus described in any one of claim 1 to claim 3, characterized by comprising a timer for measuring an elapsed time from a start of extracorporeal circulation of the blood of the patient, the replacement of the priming solution with the blood being detected, based on the elapsed time measured by the timer, in the vicinity of the end portion of the venous blood circuit on the upstream side of the first valve.

### EFFECT OF THE INVENTION

According to the invention of claim 1, since the detection device detects replacement of priming solution with blood to automatically switch the first valve from a closed state to an open state and the second valve from an open state to a closed state, an efficient blood purification treatment can be performed and a large amount of priming solution can be prevented from being introduced to the body of a patient at the time the treatment is started, and the extracorporeally-circulated blood can be efficiently returned into the patient.

According to the invention of claim 2, since the discharge line is constructed by an overflow line extending from the air-layer side of the venous drip chamber, the overflow line required in a priming operation can be also utilized in the blood purification treatment, so that use can be made of the existing constituents in an blood purification apparatus, thus suppressing the manufacturing cost.

According to the invention of claim 3, replacement of the priming solution with the blood is detected based on a pressure difference between a liquid pressure in the arterial drip chamber and a liquid pressure in the venous drip chamber in the vicinity of the end portion of the venous blood circuit, so that the replacement of the priming solution with the blood can be detected utilizing the existing constituents in an blood purification apparatus, thus suppressing the manufacturing cost.

According to the invention of claim 4, since a timer is provided for measuring an elapsed time from a start of extracorporeal circulation of the blood of a patient, and replacement of the priming solution with the blood is detected based on the elapsed time measured by the timer in the vicinity of the end portion of the venous blood circuit, replacement of the priming solution with the blood can be detected by using a simple structure, thus suppressing the manufacturing cost.

### BEST MODE OF CARRYING OUT THE INVENTION

Hereinafter, a mode of carrying-out the present invention will be explained in detail with reference to the accompanying drawings.

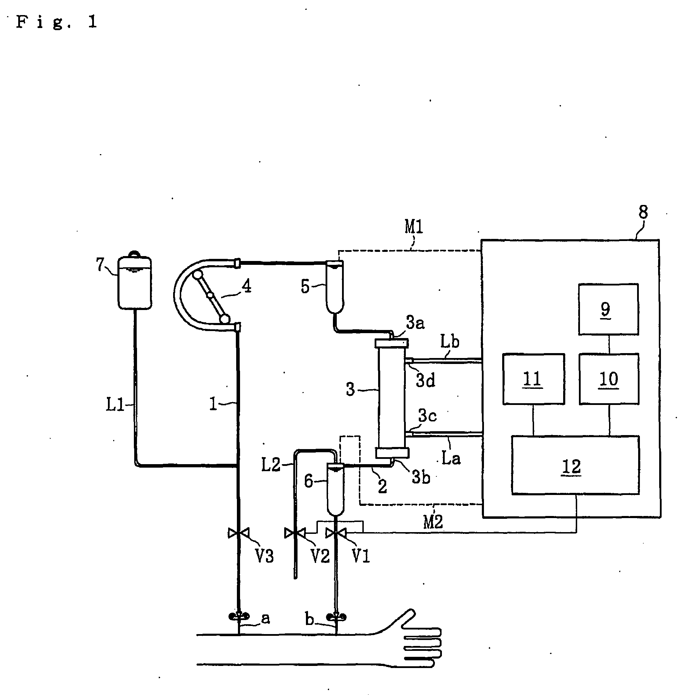

A blood purification apparatus according to the present carrying-out mode is constructed by a dialysis apparatus for performing a dialysis treatment, and as shown in Fig. 1, is mainly constructed by a blood circuit formed by an arterial blood circuit 1 and a venous blood circuit 2, a dialyzer 3 (blood purifying device) provided between the arterial blood circuit 1 and the venous blood circuit 2 for purifying the blood flowing in the blood circuit, a blood pump 4, an arterial drip chamber 5 and a venous drip chamber 6 provided in the arterial blood circuit 1 and the venous blood circuit 2, respectively, an overflow line L2 (discharge line) extending from an air-layer side of the venous drip chamber 6, and a dialysis device body 8 for supplying dialysate to the dialyzer 3.

The arterial blood circuit 1 is provided with an arterial needle "a" connected to an end portion thereof, and the blood pump 4 (roller-type pump) and the arterial drip chamber 5 for removing air-bubbles are provided in the middle thereof. The venous blood circuit 2 is provided with a venous needle "b" connected to an end portion thereof, and the venous drip chamber 6 is provided in the middle thereof When the blood pump 4 is driven while the arterial needle "a" and the venous needle "b" are inserted into the body of a patient, the blood of the patient flows through the arterial blood circuit 1 into the dialyzer 3 where the blood is purified. Then, the blood returns into the body of the patient through the venous blood circuit 2 while air-bubbles in the blood are removed in the venous drip chamber 6. Thus, the blood of the patient is purified by the dialyzer 3 during extracorporeal circulation from the end portion of the arterial blood circuit 1 to the end portion of the venous blood circuit 2.

The dialyzer 3 is provided with a blood inlet port 3a, a blood outlet port 3b, a dialysate inlet port 3c and a dialysate outlet port 3d on a body member. The arterial blood circuit 1 is connected to the blood inlet port 3 a, and the venous blood circuit 2 is connected to the blood outlet port 3b. Further, the dialysate inlet port 3c and the dialysate outlet port 3d are connected to a dialysate inlet line La and a dialysate outlet line Lb, respectively, which are extended from the dialysis device body 8.

The dialyzer 3 includes a plurality of hollow fibers. The blood flows through the inside of the hollow fibers, and the dialysate flows through a portion between the outer surfaces of the hollow fibers and the inner surface of the body member of the dialyzer 3. In the hollow fibers, a large number of micro holes (pores) penetrating between the inner and outer surfaces of the hollow fibers are formed to form permeable membranes where impurities and the like in the blood are permeated into the dialysate.

The dialysis device body 8 is provided with a water removal pump (not shown) for removing water from the blood of a patient flowing through the inside of the dialyzer 3. Additionally, one end of the dialysate inlet line La is connected to the dialyzer 3 (the dialysate inlet port 3c), and another end of the dialysate inlet line La is connected to a dialysate supplying device (not shown) for adjusting a predetermined concentration of dialysate. Also, one end of the dialysate outlet line Lb is connected to the dialyzer 3 (the dialysate outlet port 3d), and another end is connected to a fluid disposal means which is not shown. Thus, after the dialysate supplied from the dialysis supplying device flows through the dialysate inlet line La into the dialyzer 3, the dialysate is fed to the fluid disposal means through the dialysate outlet line Lb.

Extensions of the arterial drip chamber 5 and the venous drip chamber 6 are monitor tubes M1 and M2, respectively, end portions ofwhich being connected to the dialysis device body 8. Further, the dialysis device body 8 comprises a pressure sensor 9, which can measure a liquid pressure in the arterial drip chamber 5 (a blood pressure at a dialyzer inlet) and a liquid pressure in the venous drip chamber 6 (a venous blood pressure) by the monitors M1 and M2, respectively.

Further, a third electromagnetic valve V3 is provided in the vicinity of the arterial needle "a" at the arterial blood circuit 1 (between the arterial needle "a" and a joint point of a saline line L1), and a first electromagnetic valve V1 is provided in the vicinity of the venous needle "b" at the venous blood circuit 2 (the vicinity of an end portion of the venous blood circuit 2 between the venous drip chamber 6 and the venous needle "b"). The first electromagnetic valve V1 can open and close the vicinity of the venous needle "b" to shut or allow the priming solution and the blood from flowing into the patient through the venous needle "b".

Furthermore, the saline line L1 is extended from a portion of the arterial blood circuit 1 between the arterial needle "a" and the blood pump-4, and another end of the saline line L1 is connected to a saline solution bag 7 supplied with the priming solution (a saline solution). An overflow line L2 is extended from an air-layer side (an upper portion) of the venous drip chamber 6, and the middle of the overflow line L2 is provided with a second electromagnetic valve V2 which can open and close the overflow line L2 to shut or allow flows of the priming solution and the blood.

Therefore, when rinsing and priming are performed prior to the dialysis treatment, an end portion of the arterial blood circuit 1 and an end portion of the venous blood circuit 2 are connected to each other before the arterial needle "a" and the venous needle "b" are mounted thereon. Then, after the electromagnetic valves V1 to V3 are switched to an open state, and the blood pump 4 is driven to introduce the priming solution (a saline solution) from the saline solution bag 7 into the blood circuit, and then a portion of the priming solution overflowed from the venous drip chamber 6 is discharged from the overflow line L2. Accordingly, the entire blood circuit is filled with the priming solution. Note that, when the priming is finished, the electromagnetic valves V1 and V3 are switched to a closed state, and the saline line L1 is closed by using a forceps and the like.

Then, at the time the dialysis treatment is started, the arterial needle "a" and the venous needle "b" are mounted on the end portion of the arterial blood circuit 1 and the end portion of the venous blood circuit 2, respectively, and then are inserted into a patient. After that, the dialysate is supplied from the dialysis device body 8 to the dialyzer 3, and the blood pump 4 is driven to introduce the blood of a patient into the blood circuit through the arterial needle "a". Immediately after the dialysis treatment is started, the first electromagnetic valve V1 is in a closed state, and the second and third electromagnetic valves V2 and V3 are in an open state, so that the pre-filled priming solution is discharged from the overflow line L2 during the process of replacing the priming solution with the blood on an upstream side of the blood circuit.

Moreover, the dialysis device body 8 is provided with a pressure difference calculation means 10 electrically connected to the pressure sensor 9, a timer 11 which measures an elapsed time from the start of the dialysis treatment (the start of the extracorporeal circulation of the blood of a patient), and a switching control means 12 electrically connected to the pressure difference calculation means 10 and the timer 11. Note that, the pressure difference calculation means 10 and the timer 11 form a detection means in the present invention.

The pressure difference calculation means 10 calculates a pressure difference between a liquid pressure in the arterial drip chamber 5 and a liquid pressure in the venous drip chamber 6. If conditions are such that the membrane area of the dialyzer 3 is 1.5 [m²], the inner diameter of the hollow fibers is 210 [µm], a capacity (an amount of the blood filled) is 91 [mL], the inner diameter of tubes of the blood circuit is 3.4 [mm], a capacity of the arterial blood circuit 1 is 63 [mL], a capacity of the venous blood circuit 2 is 64 [mL], a temperature of the blood is 37 [°C], a hematocrit value is 32

[%], and a flow rate is 100 [mL/min], it is found that a resultant pressure difference gradually increases as time elapses as shown in Fig. 2.

That is, since the viscosity of the blood is higher than that of the priming solution, the pressure difference obtained by the pressure difference calculation means 10 has a low value while the priming solution is flowing through the dialyzer 3, and becomes a higher value due to the tube resistance as the blood starts flowing through the dialyzer 3 after the priming solution is replaced by the blood. When the replacement of the priming solution with the blood is performed, for example, a pressure difference α [mmHg] at the time the replaced blood reaches the upstream side of the dialyzer 3, a pressure difference β [mmHg] at the time the replaced blood reaches the downstream side of the dialyzer 3 and a pressure difference γ [mmHg] at the time the replaced blood is discharged from the overflow line L2 are obtained in advance, then, the replacement of the priming solution with the blood in the vicinity of the venous needle "b" (the vicinity of the end portion of the venous blood circuit 2 on the upstream side of the first electromagnetic valve V1) can be detected by detecting the moment the pressure difference obtained by the pressure difference calculation means 10 reaches β [mmHg].

The switching control means 12 is electrically connected to the first and second electromagnetic valves V1 and V2, and can switch the valves V1 and V2 to open and closed states. When the pressure difference obtained through the pressure difference calculation means 10 reaches, the switching control means 12 automatically switches the second electromagnetic valve V2 from an open state to a closed state and the first electromagnetic valve V1 from a closed state to an open state, which shuts off the discharge from the overflow line L2 and the extracorporeally-circulated blood returns into the patient through the venous needle "b".

Accordingly, in contrast to a blood purification apparatus in which the first and second valves V1 and V2 are manually switched by visually confirming that the priming solution is replaced with the blood in the vicinity of the venous needle "b" at the time the blood purification treatment is started, the operating efficiency can be improved, a large amount of priming solution can be prevented from flowing into a patient, and an extracorporeally-circulated blood can be efficiently returned into a patient. Further, in a conventional dialysis device, since liquid pressures in the arterial drip chamber 5 and the venous drip chamber 6 are measured by a pressure sensor set in the dialysis device body 8, a replacement of the priming solution with the blood can be detected based on a pressure difference by utilizing the constituents in the existing blood purification apparatus, which can suppress the manufacturing cost.

Additionally, the blood purification apparatus may include an alarm means which warns that the blood is discharged from the overflow line L2 when the pressure difference obtained by the pressure difference calculation means 10 reaches γ [mmHg]. Such alarm means may be an audible alert with a speaker, or a thing that displays alerting messages. In addition, the graph shown in Fig. 2 may be displayed to have a medical staff visually recognize a tendency in the pressure difference.

As described above, the timer 11 measures the elapsed time from the start of the dialysis treatment (i.e., the start of the extracorporeal circulation of the blood of a patient). A period of time (switching time) until the blood reaches the upstream side of the dialyzer 3 by the replacement of the priming solution with the blood is obtained in advance, so that the timer 11 transmits to the switching control means 12 a signal indicating that the switching time has passed, and then the switching control means 12 automatically switches the second electromagnetic valve V2 from the open state to the closed state and switches the first electromagnetic valve V1 from the closed state to the open state.

Accordingly, even if the pressure difference calculation means 10 does not properly perform the detection operation due to either mechanical problems or electrical problems, the timer 11 can forcibly switch the first and second electromagnetic valves V1 and V2. Thus, the timer 11 functions as a socalled fail-safe device.

Furthermore, although the present invention is applied to a dialysis device utilized in the dialysis treatment in the above-described carrying-out mode, the present invention may be applied to other devices (such as devices in hemodiafiltration or hemofiltration, blood purification apparatuses used for AFBF, and blood plasma absorption devices) for purifying the blood of a patient while extracorporeally circulating the blood.

### INDUSTRIAL POTENTIAL OF UTILIZATION

The present invention may be applied to other modes of a blood purification apparatus, if the apparatus prevents a priming solution from flowing into an end portion of the venous blood circuit and discharge the priming solution through a discharge line extending from the end portion of the venous blood circuit when the priming solution is flowing in the end portion of the venous blood circuit, and automatically prevent the flow through the discharge line from flowing through the discharge line to flowing into the end portion of the venous blood circuit when a detecting means detects that the priming solution flowing through the end portion of the venous blood circuit is replaced with the blood.

### BRIEF DESCRIPTION OF DRAWINGS

- Fig. 1: A schematic diagram illustrating a dialysis device (blood purification device) according to the mode of carrying out the present invention.
- Fig. 2: A graph where the ordinate shows a pressure difference calculated by a pressure difference calculation means in the above-dialysis device and the abscissa shows an elapsed time from the start of a dialysis treatment.
- Fig. 3: A schematic diagram illustrating a prior art dialysis device.

### DESCRIPTION OF NUMERICAL REFERENCES

1 Arterial blood circuit
2 Venous blood circuit
3 Dialyzer (Blood purification means)
4 Blood pump
5 Arterial drip chamber
6 Venous drip chamber
7 Saline solution bag
8 Dialysis device body
9 Pressure sensor
10 : Pressure difference calculation means (Detection means)
11 Timer
12 : Switching control means
   a : Arterial needle
   b : Venous needle
   L1 : Saline line
   L2 : Overflow line (discharge line)
   V1 : First electromagnetic valve (First valve)
   V2 : Second electromagnetic valve (Second valve)
   V3 : Third electromagnetic valve

## Claims

1. A blood purification apparatus comprising:
a blood circuit formed by an arterial blood circuit (1) and a venous blood circuit (2), said blood circuit being pre-filled with a priming solution in advance for extracorporeally-circulating the blood of a patient from an end portion of said arterial blood circuit (1) to an end portion of said venous blood circuit (2);
a blood purifying means (3), provided between said arterial blood circuit (1) and said venous blood circuit (2) of said blood circuit, for purifying the blood flowing through said blood circuit;
a first valve (V1) provided for opening and closing the vicinity of said end portion of said venous blood circuit (1) to prevent and allow the flow of said priming solution and said blood flowing into said patient through said end portion of said venous blood circuit (2);
a discharge line(L2) extending from an upstream side of said first valve (V1) of said blood circuit for discharging said priming solution flowing through said blood circuit; and
a second valve (V2) provided for opening and closing said discharge line (L2) to prevent and
allow the flow of said priming solution and said blood flowing through said discharge line (L2), **characterized in that**,
said vicinity of said end portion of said venous blood circuit (2) on said upstream side of said first valve (V1) is provided with a detection means for detecting a replacement of said priming solution with said blood, and the detection of said replacement by said detection means automatically switches said first valve (V1) from a closed state to an open state and said second valve (V2) from an open state to a closed state.

2. The blood purification apparatus as set forth in claim 1, **characterized in that** said arterial blood circuit (1) and said venous blood circuit (2) are provided with an arterial drip chamber (5) and a venous drip chamber (6), respectively, for removing bubbles in said priming solution and said blood, and said discharge line is constructed by an overflow line extending from an air-layer side of said venous drip chamber (6).

3. The blood purification apparatus as set forth in claim 2, **characterized in that** replacement of said priming solution with said blood is detected, based on a pressure difference between a liquid pressure in said arterial drip chamber (5) and a liquid pressure in said venous drip chamber (5), in said vicinity of said end portion of said venous blood circuit (2) on said upstream side of said first valve (V1).

4. The blood purification apparatus as set forth in any one of claim 1 to claim 3, **characterized by** comprising a timer (11) for measuring an elapsed time from a start of extracorporeal circulation of said blood of said patient, said replacement of said priming solution with said blood being detected, based on said elapsed time measured by said timer, in said vicinity of said end portion of said venous blood circuit (2) on said upstream side of said first valve (V1).

## Patentansprüche

1. Blutreinigungsvorrichtung, aufweisend:
einen Blutkreislauf, der durch einen arteriellen Blutkreislauf (1) und einen venösen Blutkreislauf (2) gebildet ist, wobei der Blutkreislauf mit einer Primer-Lösung vorab vorbefüllt wird, um das Blut eines Patienten extrakorporal von einem Endabschnitt des arteriellen Blutkreislaufes (1) zu einem Endabschnitt des venösen Blutkreislaufes (2) zu zirkulieren;
eine Blutreinigungseinrichtung (3), die zwischen dem arteriellen Blutkreislauf (1) und
dem venösen Blutkreislauf (2) des Blutkreislaufes vorgesehen ist, um das durch den Blutkreislauf strömende Blut zu reinigen;
ein erstes Ventil (V1), das vorgesehen ist, um die Umgebung des Endabschnittes des venösen Blutkreislaufes (1) zu öffnen und zu schließen, um das Strömen der Primer-Lösung und des Blutes zu verhindern und zu gestatten, und zwar beim Einströmen in den Patienten durch den Endabschnitt des venösen Blutkreislaufes (2);
eine Abführleitung (L2), die sich von einer zulaufseitigen Seite des ersten Ventils (V1) des Blutkreislaufes erstreckt, um die durch den Blutkreislauf strömende Primer-Lösung abzuführen; und
ein zweites Ventil (V2), das vorgesehen ist, um die Abführleitung (L2) zu öffnen und zu schließen, um das Strömen der Primer-Lösung und des Blutes zu verhindern und zu gestatten, die durch die Abführleitung (L2) strömen, **dadurch gekennzeichnet, dass**
die Umgebung des Endabschnittes des venösen Blutkreislaufes (2) auf der zulaufseitigen Seite des ersten Ventils (V1) mit einer Erfassungseinrichtung versehen ist, um einen Austausch der Primer-Lösung gegen das Blut zu erfassen, und bei Erfassen des Austausches durch die Erfassungseinrichtung automatisch das erste Ventil (V1) aus einem geschlossenen Zustand in einen offenen Zustand und das zweite Ventil (V2) aus einem offenen Zustand in einen geschlossenen Zustand umgeschaltet wird.

2. Blutreinigungsvorrichtung nach Anspruch 1, **dadurch gekennzeichnet, dass** der arterielle Blutkreislauf (1) und der venöse Blutkreislauf (2) mit einer arteriellen Tropfkammer (5) bzw. einer venösen Tropfkammer (6) versehen sind, um Blasen in der Primer-Lösung und dem Blut zu entfernen, und die Abführleitung durch eine Überlaufleitung aufgebaut ist, die sich vonseiten der Luftschicht der venösen Tropfkammer (6) erstreckt.

3. Blutreinigungsvorrichtung nach Anspruch 2, **dadurch gekennzeichnet, dass** der Austausch der Primer-Lösung gegen das Blut basierend auf einer Druckdifferenz zwischen einem Flüssigkeitsdruck in der arteriellen Tropfkammer (5) und einem Flüssigkeitsdruck in der venösen Tropfkammer (5) erfasst wird, und zwar in der Nähe des Endabschnittes des venösen Blutkreislaufes (2) auf der zulaufseitigen Seite des ersten Ventils (V1).

4. Blutreinigungsvorrichtung nach einem der Ansprüche 1 bis 3, **dadurch gekennzeichnet, dass** sie einen Zeitgeber (11) aufweist, um eine verstrichene Zeit von einem Anfang einer extrakorporalen Zirkulation des Blutes des Patienten zu messen, wobei der Austausch der Primer-Lösung gegen das Blut basierend auf der durch den Zeitgeber gemessenen verstrichenen Zeit erfasst wird, und zwar in der Nähe des Endabschnittes des venösen Blutkreislaufes (2) auf der zulaufseitigen Seite des ersten Ventils (V1).

## Revendications

1. Appareil de purification du sang comprenant :
un circuit de sang formé par un circuit de sang artériel (1) et un circuit de sang veineux (2), ledit circuit de sang étant prérempli avec une solution d'amorce à l'avance pour une circulation extracorporelle du sang d'un patient d'une portion d'extrémité dudit circuit de sang artériel (1) vers une portion d'extrémité dudit circuit de sang veineux (2) ;
un moyen de purification du sang (3), prévu entre ledit circuit de sang artériel (1) et ledit circuit de sang veineux (2) dudit circuit de sang, pour purifier le sang s'écoulant à travers ledit circuit de sang ;
une première valve (V1) prévue pour ouvrir et fermer le voisinage de ladite portion d'extrémité dudit circuit de sang veineux (1) afin d'empêcher et de permettre l'écoulement de ladite solution d'amorce et dudit sang s'écoulant dans ledit patient à travers ladite portion d'extrémité dudit circuit de sang veineux (2) ;
une ligne de décharge (L2) s'étendant à partir d'un côté amont de ladite première valve (V1) dudit circuit de sang pour décharger ladite solution d'amorce s'écoulant à travers ledit circuit de sang ; et
une seconde valve (V2) prévue pour ouvrir et fermer ladite ligne de décharge (L2) afin d'empêcher et de permettre l'écoulement de ladite solution d'amorce et dudit sang s'écoulant à travers ladite ligne de décharge (L2), **caractérisé en ce que**
ledit voisinage de ladite portion d'extrémité dudit circuit de sang veineux (2) sur ledit côté amont de ladite première valve (V1) est prévu avec un moyen de détection pour détecter un remplacement de ladite solution d'amorce par ledit sang, et la détection dudit remplacement par ledit moyen de détection commute automatiquement ladite première valve (V1) d'un état fermé à un état ouvert et ladite seconde valve (V2) d'un état ouvert à un état fermé.

2. Appareil de purification du sang selon la revendication 1, **caractérisé en ce que** ledit circuit de sang artériel (1) et ledit circuit de sang veineux (2) sont prévus avec une chambre de goutte-à-goutte artériel (5) et une chambre de goutte-à-goutte veineux (6), respectivement, pour éliminer les bulles dans ladite solution d'amorce et ledit sang, et ladite ligne de décharge est construite par une ligne de débordement s'étendant à partir d'un côté d'aération de ladite chambre de goutte-à-goutte veineux (6).

3. Appareil de purification du sang selon la revendication 2, **caractérisé en ce que** le remplacement de ladite solution d'amorce par ledit sang est détecté, d'après une différence de pression entre une pression de liquide dans ladite chambre de goutte-à-goutte artériel (5) et une pression de liquide dans ladite chambre de goutte-à-goutte veineux (6), audit voisinage de ladite portion d'extrémité dudit circuit de sang veineux (2) sur ledit côté amont de ladite première valve (V1).

4. Appareil de purification du sang selon l'une quelconque des revendications 1 à 3, **caractérisé en ce qu'**il comprend un minuteur (11) pour mesurer un temps écoulé à partir du début d'une circulation extracorporelle dudit sang dudit patient, ledit remplacement de ladite solution d'amorce par ledit sang étant détecté, d'après ledit temps écoulé mesuré par ledit minuteur, audit voisinage de ladite portion d'extrémité dudit circuit de sang veineux (2) sur ledit côté amont de ladite première valve (V1).
